Europäisches Patentamt

European Patent Office

Office européen des brevets

(11) Veröffentlichungsnummer: **0 014 765**
**B1**

(12) EUROPÄISCHE PATENTSCHRIFT

(45) Veröffentlichungstag der Patentschrift:
23.09.81

(51) Int. Cl.³: **C 07 C 13/465**, C 07 C 2/42

(21) Anmeldenummer: **79105238.4**

(22) Anmeldetag: **18.12.79**

(54) Verfahren zur Herstellung von 1-Phenyl-1.3.3-trimethylindan.

(30) Priorität: **19.02.79 DE 2906294**

(43) Veröffentlichungstag der Anmeldung:
**03.09.80 Patentblatt 80/18**

(45) Bekanntmachung des Hinweises auf die Patenterteilung:
**23.09.81 Patentblatt 81/38**

(84) Benannte Vertragsstaaten:
**AT BE FR GB IT NL SE**

(56) Entgegenhaltungen:
**DE-A-2 029 026**
**US-A-3 161 692**
**CHEMICAL ABSTRACTS, Band 78, Nr. 21, 28. Mai**
**1973, Seite 352, Nr. 135 935q, Columbus, Ohio, USA**

**CHEMICAL ABSTRACTS, Band 84, Nr. 23, 7. Juni**
**1976, Seite 442, Nr. 164 523h, Columbus, Ohio, USA**

(73) Patentinhaber: **CHEMISCHE WERKE HÜLS AG,**
**Postfach 1320, D-4370 Marl 1 (DE)**

(72) Erfinder: **Ratajczak, Hans-Josef, Dr., Gersthofener**
**Strasse 14, D-4370 Marl (DE)**

## Verfahren zur Herstellung von 1-Phenyl-1.3.3-trimethylindan

Es ist bekannt, daß α-Methylstyrol in Gegenwart saurer Katalysatoren dimerisiert werden kann. Je nach Art der Reaktionsbedingungen und des Katalysators werden ungesättigte offenkettige Dimere oder das gesättigte cyclische Dimere (Indan-Derivat) erhalten.

Bevorzugte Verfahren zur Herstellung des gesättigten Dimeren sind durch den Einsatz von konzentrierter Schwefelsäure als Katalysator gekennzeichnet (Organic Syntheses, Coll. Vol. 1 [1963], Seite 665; DE-OS 26 59 597 = GB-PS 1 507 600 = US-PS 4 081 489). Dabei werden jedoch, neben erheblichen Korrosionsproblemen, zufriedenstellende Produkte nur über ein aufwendiges Reinigungsverfahren erhalten.

Zur Vermeidung obiger Nachteile wird in der US-PS 3 161 692 die Verwendung von mit Säure vorbehandelter Montmorrilonit-Erde als Katalysator vorgeschlagen. Der Einsatz der dort beschriebenen Katalysatoren erfordert allerdings recht hohe Temperaturen und relativ lange Reaktionszeiten. Gleichzeitig sind die Katalysator-Lebenszeiten bzw. -Leistungen wenig zufriedenstellend.

Aufgabe der vorliegenden Erfindung ist daher ein Verfahren zur Dimerisierung von α-Methylstyrol, das bei niedrigen Temperaturen, kurzen Reaktionszeiten und hohen Katalysator-Lebenszeiten in guten Ausbeuten zum 1-Phenyl-1.3.3-trimethylindan führt.

Die Aufgabe wurde dadurch gelöst, daß man α-Methylstyrol in reiner Form oder in einem geeigneten inerten Lösemittel kontinuierlich oder diskontinuierlich in Rührbehältern bei Temperaturen von 60 bis 140° C und Verweilzeiten von 5 bis 200 Minuten mit einem Katalysator in Berührung bringt, der in an sich bekannter Weise erhalten wird, indem man getrockneten und gepulverten Bentonit (montmorillonithaltigen Ton) mit konzentrierter Schwefelsäure ohne ein nachfolgendes Auswaschen behandelt, die Schwefelsäure in einer Menge anwendet, die der Ionenaustauschfähigkeit des Bentonits gleichkommt oder um den Faktor 0,5 bis 1,5 abweicht und diesen Katalysator in einer Menge von 0,5 bis 50 Gewichtsprozent, bezogen auf das Reaktionsgemisch, einsetzt. Besonders geeignet sind Katalysatoren, die gemäß der DE-PS 10 69 583 hergestellt werden.

Zur Durchführung der Dimerisierung kann der Katalysator in einem Kolben vorgelegt und das α-Methylstyrol hinzugegeben werden. Der Katalysator kann auch zum α-Methylstyrol gegeben werden.

Die Reaktionstemperatur liegt bei 60 bis 140° C, bevorzugt bei 80 bis 120° C.

Wegen der positiven Wärmetönung der Reaktion ist es günstig, das α-Methylstyrol mengengeregelt zum Katalysator zu geben, daß die Temperatur im angegebenen Bereich gehalten werden kann.

Zur leichteren Reaktionsführung kann der Katalysator in einem inerten Lösemittel suspendiert werden (z. B. Benzol, Toluol, Cumol, CCl₄, 1-Phenyl-1.3.3-trimethylindan). Gegebenenfalls kann auch das α-Methylstyrol, verdünnt mit einem inerten Lösemittel, zugegeben werden.

Die Katalysatormenge, bezogen auf das Reaktionsgemisch, beträgt 0,5 bis 50 Gewichtsprozent. Als Reaktionsgemisch ist hier das zum Ende der Umsetzung vorliegende Reaktionsgemisch zu verstehen. In einer bevorzugten Ausführungsform wird der Katalysator in Staubform eingesetzt, in einer Korngröße unter 100 μ. Geformte Kontakte (Kugeln, Strangpreßlinge) zeigen eine deutlich geringere Aktivität und Selektivität.

Überraschend bei Einsatz obiger Katalysatoren ist die große Reaktionsgeschwindigkeit bei vergleichsweise niedrigen Temperaturen. Bereits wenige Sekunden nach beendeter Zugabe von α-Methylstyrol kann kein Monomeres mehr im Reaktionsgemisch nachgewiesen werden.

Bei Einsatz von Schwefelsäure oder den in der US-PS 3 161 692 beschriebenen Katalysatoren muß der Ansatz dagegen einige Stunden bei deutlich höheren Temperaturen nachreagieren.

Besonders überraschend ist, daß im Rührgefäß mit kontinuierlichem Zu- und Ablauf auch bei sehr kurzen mittleren Verweilzeiten (etwa 5 bis 60 Minuten) vollständiger Umsatz erzielt wird. Angesichts des bekannten Verweilzeitspektrums für den einfachen Rührkessel ist das nur durch eine außergewöhnlich hohe Reaktionsgeschwindigkeit zu erklären. Bei dieser kontinuierlichen Reaktionsführung wird das Produkt über eine geeignete Filtervorrichtung entnommen, so daß der Katalysator im Gefäß zurückgehalten wird. Der Stand im Gefäß wird dabei über eine entsprechende Regelung gehalten. Als Edukt können Mischungen aus α-Methylstyrol und geeigneten Lösemitteln (im beliebigen Verhältnis) aber auch reines α-Methylstyrol eingesetzt werden. Im letzteren Fall müssen die Abgangsleitungen beheizt sein, da das Produkt beim Abkühlen auskristallisiert.

Die mittlere Verweilzeit liegt bei 5 bis 200 Minuten, bevorzugt bei 10 bis 100 Minuten.

Die Katalysator-Leistung (kg Produkt/kg Katalysator) ist sehr groß, über 2000. Bei einem Wert von 2000 ist noch keine Desaktivierung des Kontaktes festzustellen.

Insgesamt stellt die vorliegende Erfindung, insbesondere die kontinuierliche Variante, ein äußerst einfaches und entsprechend kostengünstiges Verfahren zur Herstellung von 1-Phenyl-1.3.3-trimethylindan dar. Es ist in der Reaktionsführung, der Raum-Zeit-Ausbeute und der Katalysator-Leistung den bekannten Verfahren weit überlegen.

Bei Verwendung eines Lösemittels kann dieses leicht durch Destillation (z. B. am Dünnschichtverdampfer) entfernt werden. Das Produkt selber fällt in so reiner Form an, daß eine Destillation für die meisten Verwendungszwecke entfällt.

### Beispiele 1 bis 4

In einem 500-ml-Dreihalskolben, der mit Magnetrührer, Tropftrichter, Thermometer und Kühler versehen war, wurde der Katalysator, suspendiert in 50 ml Cumol, vorgelegt. Es wurden ausschließlich Katalysatoren der K-Serie der Süd-Chemie AG (München) eingesetzt, die gemäß der DE-PS 10 69 583 hergestellt werden.

Der Kolben wurde auf Reaktionstemperatur erhitzt und das α-Methylstyrol, gegebenenfalls in einem Lösemittel, zugetropft. Etwa 5 Minuten nach Beendigung des Zutropfens wurde der Rührer abgestellt und nach Absitzen des Katalysators eine Probe gezogen. Nach Zugabe eines geeigneten Standards (z. B. Diphenyl) wurde die Probe durch Gaschromatographie und Hochdruckflüssigkeitschromatographie analysiert. Die HPLC-Analyse ist erforderlich, da neben dem Dimeren in kleinen Mengen noch ein Trimeres entsteht (identifiziert durch MS-Analyse), das gaschromatographisch nicht nachgewiesen werden kann.

Die Versuchsbedingungen und Ergebnisse der einzelnen Versuche sind in der Tabelle 1 zusammengestellt.

### Beispiele 5 bis 9

Zur kontinuierlichen Versuchsdurchführung wurde der Katalysator, suspendiert in 50 ml Cumol, in einem 500-ml-Dreihalskolben mit Magnetrührer und Thermometer vorgelegt. Nach Aufheizen auf Reaktionstemperatur wurde das α-Methylstyrol bzw. α-Methylstyrol/Lösungsmittelgemisch mittels einer Pumpe über ein Tauchrohr mit Schliff in den Kolben gepumpt. Nach Erreichen eines bestimmten Füllstandes (300 ml) wurde der Kolben dicht verschlossen und das Proudkt über eine, an ein Glasrohr mit Schliff angeschmolzene Filterkerze, die in das Reaktionsgemisch eintauchte, kontinuierlich, entsprechend der Zulaufmenge aufgenommen. Verstopfungen an der Filterkerze wurden nie beobachtet. Die Proben wurden nach einer Laufzeit von jeweils 30 Stunden gezogen. Die Analytik wurde analog den Beispielen 1 bis 5 durchgeführt. Die Versuchsbedingungen und Ergebnisse sind in der Tabelle 2 dargestellt.

Die Ergebnisse eines Langzeitversuches sind in der Tabelle 3 aufgeführt.

Tabelle 1

Dimerisierung von α-Methylstyrol (diskontinuierlich)

| Beispiel | Kataly-sator | Menge Kat. (g) | α-Methyl-styrol (g) | Cumol (g) | Zutropf-zeit (Min.) | Temp. (°C) | Umsatz (%) | Ausbeute Dimeres | Trimeres |
|---|---|---|---|---|---|---|---|---|---|
| 1 | KSF/O*) | 5 | 250 | — | 60 | 100 | 100 | 93 | 6 |
| 2 | KSF/O | 10 | 125 | 12⁵ | 25 | 100 | 100 | 95 | 4 |
| 3 | KSF/O | 20 | 200 | 100 | 30 | 80 | 100 | 94 | 5 |
| 4 | KSF/O | 30 | 125 | 125 | 10 | 105 | 100 | 95 | 4 |

*) Montmorillonit, $SiO_2$ (α-Quarz), behandelt in getrockneter und gepulverter Form mit konzentrierter Schwefelsäure ohne nachfolgendes Auswaschen gemäß DE-PS 10 69 583
innere Oberfläche 180 bis 220 $m^2$/g
$CCl_4$-Porenvolumen in $cm^3$/g: 0,38 bis 800 Å
Schüttgewicht: 350 bis 400 g/l

Tabelle 2

Dimerisierung von $\alpha$-Methylstyrol (kontinuierlich)

| Beispiel | Katalysator | Menge Kat. (g) | $\alpha$-Methylstyrol pro Stunde (g/h) | Cumol pro Std. (g/h) | Mittlere Verweilzeit (Min.) | Temp. (°C) | Umsatz (%) | % Ausbeute Dimeres | % Ausbeute Trimeres |
|---|---|---|---|---|---|---|---|---|---|
| 5 | KSF/O | 10 | 30 | 270 | 60 | 100 | 100 | 93 | 6 |
| 6 | KSF/O | 30 | 180 | 120 | 60 | 100 | 100 | 92 | 7 |
| 7 | KSF/O | 30 | 900 | 600 | 12 | 100 | 100 | 93 | 7 |
| 8 | KSF/O | 30 | 300 | — | 60 | 95 | 100 | 91 | 8 |
| 9 | KSF/O | 40 | 90 | 210 | 60 | 70 | 100 | 95 | 3 |

Tabelle 3

Langzeitversuch zur kontinuierlichen Dimerisierung von $\alpha$-Methylstyrol
Bedingungen entsprechend Beispiel 6

| Laufzeit (Tage) | Kat.-Leistung (kg Produkt/kg Kat.) | Umsatz (%) | % Ausbeute Dimeres | % Ausbeute Trimeres |
|---|---|---|---|---|
| 1 | 144 | 100 | 92 | 8 |
| 6 | 864 | 100 | 94 | 5 |
| 15 | 2160 | 100 | 94 | 5 |

**Patentansprüche**

1. Verfahren zur Herstellung von 1-Phenyl-1.3.3-trimethylindan durch Dimerisierung von $\alpha$-Methylstyrol in Gegenwart saurer Katalysatoren, dadurch gekennzeichnet, daß man $\alpha$-Methylstyrol in reiner Form oder in einem inerten Lösemittel bei Temperaturen von 60 bis 140° C und Kontaktzeiten von 5 bis 200 Minuten mit einem Katalysator in Berührung bringt, der in an sich bekannter Weise erhalten wird, indem man getrockneten und gepulverten Bentonit mit konzentrierter Schwefelsäure ohne ein nachfolgendes Auswaschen behandelt und die Schwefelsäure in einer Menge anwendet, die der Ionenaustauschfähigkeit des Bentonits gleichkommt oder um den Faktor 0,5 bis 1,5 abweicht und diesen Katalysator in einer Menge von 0,5 bis 50 Gewichtsprozent, bezogen auf das Reaktionsgemisch, einsetzt.

2. Verfahren nach Anspruch 1, dadurch gekennzeichnet, daß man die Reaktion in einem Rührgefäß mit kontinuierlichem Zu- und Ablauf durchführt, wobei der Katalysator über eine geeignete Filtervorrichtung im Gefäß zurückgehalten wird.

3. Verfahren nach den Ansprüchen 1 und 2, dadurch gekennzeichnet, daß man den Katalysator in Staubform in einer Korngröße von unter 100 µ einsetzt.

4. Verfahren nach den Ansprüchen 1 bis 3, dadurch gekennzeichnet, daß man als Lösemittel Benzol, Toluol, Cumol, CCl$_4$ und/oder 1-Phenyl-1.3.3-trimethylindan verwendet.

**Claims**

1. A process for the manufacture of 1-phenyl-1,3,3-trimethylindan by dimerization of $\alpha$-methylstyrene in the presence of acid catalysts, characterized in that $\alpha$-methylstyrene in a pure state or dissolved in an inert solvent is brought into contact with a catalyst at a temperature of 60—140° C for a contact period of 5—200 minutes, the catalyst being obtained in the usual manner by treating dried and pulverized bentonite with concentrated sulphuric acid without subsequent scrubbing, the amount of sulphuric acid being 0.5—1.5 equivalents per equivalent of bentonite based on the ion exchange capacity of the bentonite, and the amount of catalyst being 0.5—50% by weight, based on the total weight of the reaction mixture.

2. The process of Claim 1, wherein the reaction is conducted in an agitated vessel with continuous feed and discharge, the catalyst being retained in the vessel by use of a filtering device.

3. The process of Claim 1 and 2, wherein the catalyst is in dust form of a particle size of less than 100 µ.

4. The process of Claim 1—3, wherein benzene, toluene, cumene, CCl₄ and/or 1-phenyl-1,3,3-trimethylindan are used as a solvent.

## Revendications

1. Procédé pour la préparation de phényl-1-triméthyl-1,3,3-indane par dimérisation du α-méthylstyrène en présence de catalyseurs acides, caractérisé par le fait qu'on met en contact le α-méthylstyrène à l'état pour ou dans un solvant inerte, à des températures de 60 à 140°C et avec des durées de contact de 5 à 200 minutes, avec un catalyseur obtenu de manière connue, selon laquelle on traite de la bentonite séchée et à l'état de poudre avec de l'acide sulfurique concentré, sans effectuer ensuite de rinçage, la quantité d'acide sulfurique étant telle qu'elle s'équilibre avec la capacité d'échange ionique de la bentonite ou qu'elle s'en écarte d'un facteur compris entre 0,5 et 1,5, et qu'on utilise une quantité de 0,5 à 50% en poids, rapportée au mélange réactionnel, de ce catalyseur.

2. Procédé selon la revendication 1, caractérisé par le fait qu'on effectue la réaction dans un récipient avec agitation avec un apport et un écoulement continu, le catalyseur étant retenu dans le récipient par un dispositif filtre approprié.

3. Procédé selon les revendications 1 et 2, caractérisé par le fait qu'on utilise le catalyseur sous forme pulvérulente avec une granulométrie inférieure à 100 µ.

4. Procédé selon les revendications 1 à 3, caractérisé par le fait qu'on emploie comme solvant le benzène, le toluène, le cumène, le CCl₄ et/ou le phényl-1-triméthyl-1,3,3-indane.